(19) Europäisches Patentamt / European Patent Office / Office européen des brevets

(11) **EP 2 499 982 B1**

(12) **EUROPEAN PATENT SPECIFICATION**

(45) Date of publication and mention of the grant of the patent:
**25.02.2015 Bulletin 2015/09**

(51) Int Cl.:
*A61B 18/12* (2006.01)   *A61B 18/18* (2006.01)
*A61B 18/00* (2006.01)

(21) Application number: **12001841.1**

(22) Date of filing: **19.03.2012**

(54) **Energy-based ablation completion algorithm**

Energiebasierter Ablationsvervollständigungsalgorithmus

Algorithme d'exécution d'ablation fonctionnant à l'énergie

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.03.2011 US 201113050729**

(43) Date of publication of application:
**19.09.2012 Bulletin 2012/38**

(73) Proprietor: **Covidien LP**
**Mansfield, MA 02048 (US)**

(72) Inventors:
• **Ladtkow, Casey M**
  **Erie, CO 80516 (US)**
• **Brannan, Joseph D**
  **Erie, CO 80516 (US)**

(74) Representative: **Hoffmann Eitle**
**Patent- und Rechtsanwälte PartmbB**
**Arabellastraße 30**
**81925 München (DE)**

(56) References cited:
**US-B1- 6 575 969    US-B2- 6 962 587**

**Description**

Technical Field

The present disclosure relates to electrosurgical apparatuses, systems and methods. More particularly, the present disclosure is directed to electrosurgical systems and methods for monitoring electrosurgical procedures and intelligent termination thereof based on various sensed tissue parameters.

Background of Related Art

Energy-based tissue treatment is well known in the art. Various types of energy (e.g., electrical, ohmic, resistive, ultrasonic, microwave, cryogenic, laser, etc.) are applied to tissue to achieve a desired result. Electrosurgery involves application of high radio frequency electrical current to a surgical site to cut, ablate, coagulate or seal tissue. In monopolar electrosurgery, a source or active electrode delivers radio frequency energy from the electrosurgical generator to the tissue and a return electrode carries the current back to the generator. In monopolar electrosurgery, the source electrode is typically part of the surgical instrument held by the surgeon that is applied to the tissue. A patient return electrode is placed remotely from the active electrode to carry the current back to the generator.

Ablation is most commonly a monopolar procedure that is particularly useful in the field of cancer treatment, where one or more RF ablation needle electrodes (usually of elongated cylindrical geometry) are inserted into a living body. A typical form of such needle electrodes incorporates an insulated sheath disposed over an exposed (uninsulated) tip. When the RF energy is provided between the return electrode and the inserted ablation electrode, RF current flows from the needle electrode through the body. Typically, the current density is very high near the tip of the needle electrode, which tends to heat and destroy surrounding issue.

In bipolar electrosurgery, one of the electrodes of the hand-held instrument functions as the active electrode and the other as the return electrode. The return electrode is placed in close proximity to the active electrode such that an electrical circuit is formed between the two electrodes (e.g., electrosurgical forceps). In this manner, the applied electrical current is limited to the body tissue positioned between the electrodes. When the electrodes are sufficiently separated from one another, the electrical circuit is open and thus inadvertent contact with body tissue with either of the separated electrodes prevents the flow of current.

Bipolar electrosurgical techniques and instruments can be used to coagulate blood vessels or tissue, e.g., soft tissue structures, such as lung, brain and intestine. A surgeon can either cauterize, coagulate/desiccate and/or simply reduce or slow bleeding, by controlling the intensity, frequency and duration of the electrosurgical energy applied between the electrodes and through the tissue. In order to achieve one of these desired surgical effects without causing unwanted charring of tissue at the surgical site or causing collateral damage to adjacent tissue, e.g., thermal spread, it is necessary to control the output from the electrosurgical generator, e.g., power, waveform, voltage, current, pulse rate, etc.

It is known that measuring the electrical impedance and changes thereof across the tissue at the surgical site provides a good indication of the state of desiccation or drying of the tissue, e.g., as the tissue dries or loses moisture, the impedance across the tissue rises. This observation has been utilized in some electrosurgical generators to regulate the electrosurgical power based on measured tissue impedance. US 6,962,587 discloses a method for detecting and treating tumors using impedance measurement, in which a sensing array comprising impedance sensors monitors impedances of an ablated site throughout the ablation procedure. Because impedance response is different at different frequencies and at different times throughout the ablation procedure depending on the state of the tissue being ablated, monitoring the impedances at several applied frequencies and comparing them to predicted impedance responses results in being able to estimate the relative time position of the ablation procedure, as well as being able to estimate when the ablation procedure is complete. A display may display tumor and estimated ablation volume images, time temperature profiles, and ablation setting information such as power settings and delivery time. A second mode of the system may be entered when there is a change in a slope of RF power or system impedances beyond a preset amount. When it is determined that the ablation procedure is complete, based on the estimated amount of destroyed tissue, ablation is terminated, or an alarm is displayed.

US 6,575,969 discloses an electrode system for tumor ablation having an ultrasonic scanner for imaging a portion of the body to be ablated, in which ultrasonic, sonic, CT, MRI, or PET imaging is used to visualize the real-time change in ablated tissue. Parameters such as tip temperature and power can be modified by an operator as desired, based on information obtained by viewing the displayed real-time progress of the ablation procedure.

SUMMARY

An electrosurqical generator according to independent claim 1 is provided. The generator may include sensor circuitry configured to measure voltage and current delivered to tissue and a controller configured to measure time of energy delivery to tissue and to calculate energy delivered to tissue, the controller further configured to estimate a size of an ablation volume as a function of energy delivered to tissue and time and to calculate a growth rate of the ablation volume based on the estimated size.

A method for ablating tissue is also provided by the present disclosure as explanation of the present invention. The method includes: measuring time of energy delivery to tissue; calculating energy delivered to tissue based on measured voltage and current; estimating a size of an ablation volume as a function of energy delivered to tissue and time; and calculating a growth rate of the ablation volume based on the estimated size.

A method of ablating tissue is also provided by the present disclosure as explanation of the present invention. The method includes: applying at least one electrosurgical waveform to tissue in a pulsitile manner; measuring reactive impedance of the tissue; measuring time of energy delivery to tissue; determining peaks of the reactive impedance corresponding to the pulses of the at least one electrosurgical waveform; calculating a growth rate of the ablation volume based on the estimated size.

BRIEF DESCRIPTION OF THE DRAWINGS

Various embodiments of the present disclosure are described herein with reference to the drawings wherein:

Fig. 1A          is a schematic block diagram of a monopolar electrosurgical system according to one embodiment of the present disclosure;

Fig. 1B          is a schematic block diagram of a bipolar electrosurgical system according to one embodiment of the present disclosure;

Fig. 2           is a schematic block diagram of a generator according to an embodiment of the present disclosure;

Fig. 3           is a plot of power with respect to time of a pulsatile application of electrosurgical energy according to an embodiment of the present disclosure;

Fig. 4           is a graphical representation of a linearization of energy and time plots according to an embodiment of the present disclosure;

Fig. 5           is a flow chart diagram of a method according to an embodiment of the present disclosure;

Figs. 6A-C       are plots of temperature with respect to distance from an electrode according to an embodiment of the present disclosure;

Fig. 7           is a flow chart diagram of a method according to an embodiment of the present disclosure;

Fig. 8           is a plot of reactive impedance of tissue and ablation size during application of electrosurgical energy according to one embodiment of the present disclosure; and

Fig. 9           is a flow chart diagram of a method according to an embodiment of the present disclosure.

DETAILED DESCRIPTION

Particular embodiments of the present disclosure are described hereinbelow with reference to the accompanying drawings. In the following description, well-known functions or constructions are not described in detail to avoid obscuring the present disclosure in unnecessary detail.

The generator according to the present disclosure can perform monopolar and bipolar electrosurgical procedures as well as microwave ablation procedures, including vessel sealing procedures. The generator may include a plurality of outputs for interfacing with various electrosurgical instruments (e.g., a monopolar active electrode, return electrode, bipolar electrosurgical forceps, footswitch, etc.). Further, the generator includes electronic circuitry configured for generating radio frequency power specifically suited for various electrosurgical modes (e.g., cutting, blending, division, etc.) and procedures (e.g., monopolar, bipolar, vessel sealing).

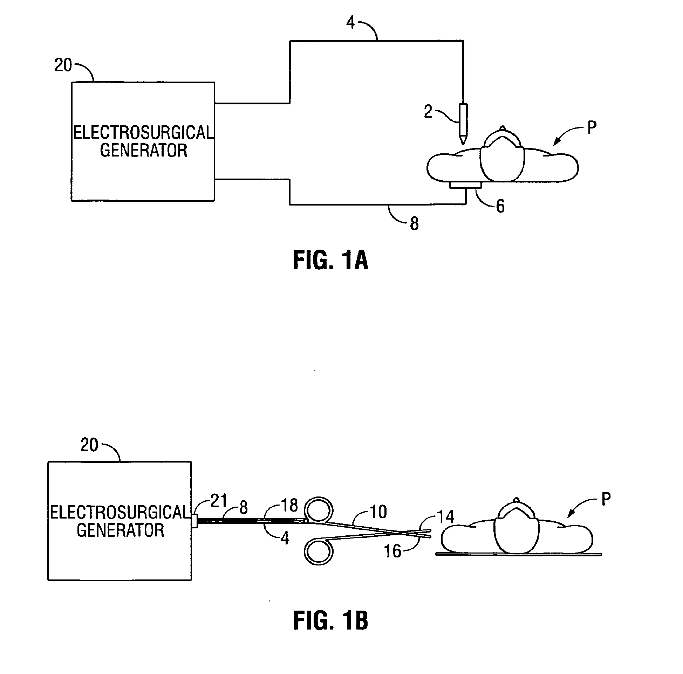

Fig. 1A is a schematic illustration of a monopolar electrosurgical system according to one embodiment of the present disclosure. The system includes an electrosurgical instrument 2 having one or more electrodes for treating tissue of a patient P. The instrument 2 is a monopolar type instrument including one or more active electrodes (e.g., electrosurgical cutting probe, ablation electrode(s), etc.). In embodiments, the instrument 2 may include a closed-loop fluid circulation mechanism coupled to a fluid circulation system that circulates a coolant fluid through one or more lumens disposed at least within a portion of the length of the active needle electrode.

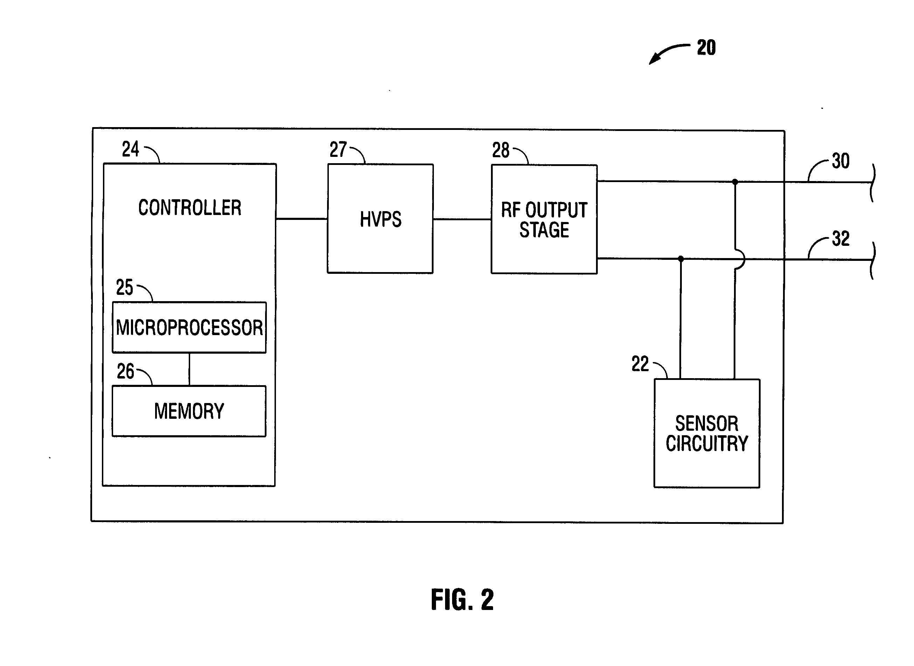

Electrosurgical RF energy is supplied to the instrument 2 by a generator 20 via a supply line 4, which is connected to an active terminal 30 (Fig. 2) of the generator 20, allowing the instrument 2 to coagulate, seal, ablate and/or otherwise treat tissue. The energy is returned to the generator 20 through a return electrode 6 via a return line 8 at a return terminal 32 (Fig. 2) of the generator 20. The active terminal 30 and the return terminal 32 are connectors configured to interface with plugs (not explicitly shown) of the instrument 2 and the return electrode 6, which are disposed at the ends of the supply line 4 and the return line 8, respectively.

The system may include a plurality of return electrodes 6 that are arranged to minimize the chances of tissue damage by maximizing the overall contact area with the patient P. In addition, the generator 20 and the return electrode 6 may be configured for monitoring so-called "tissue-to-patient" contact to insure that sufficient contact exists therebetween to further minimize the chances of tissue damage.

Fig. 1B is a schematic illustration of a bipolar electrosurgical system according to the present disclosure. The system includes a bipolar electrosurgical forceps 10 having one or more electrodes for treating tissue of a patient P. The electrosurgical forceps 10 includes opposing jaw members having an active electrode 14 and a return electrode 16 disposed therein. The active electrode 14 and the return electrode 16 are connected to the generator 20 through cable 18, which includes the supply and return lines 4, 8 coupled to the active and return terminals 30, 32, respectively (Fig. 2). The electrosurgical forceps 10 is coupled to the generator 20 at a connector 21 having connections to the active and return terminals 30 and 32 (e.g., pins) via a plug disposed at the end of the cable 18, wherein the plug includes contacts from the supply and return lines 4, 8.

The generator 20 includes suitable input controls (e.g., buttons, activators, switches, touch screen, etc.) for controlling the generator 20. In addition, the generator 20 may include one or more display screens for providing the user with variety of output information (e.g., intensity settings, "treatment complete" indicators, etc.). The controls allow the user to adjust power of the RF energy, waveform parameters (e.g., crest factor, duty cycle, etc.), and other parameters to achieve the desired waveform suitable for a particular task (e.g., coagulating, tissue sealing, intensity setting, etc.). The instrument 2 may also include a plurality of input controls that may be redundant with certain input controls of the generator 20. Placing the input controls at the instrument 2 allows for easier and faster modification of RF energy parameters during the surgical procedure without requiring interaction with the generator 20.

Fig. 2 shows a schematic block diagram of the generator 20 having a controller 24, a high voltage DC power supply 27 ("HVPS") and an RF output stage 28. The HVPS 27 is connected to a conventional AC source (e.g., electrical wall outlet) and provides high voltage DC power to an RF output stage 28, which then converts high voltage DC power into RF energy and delivers the RF energy to the active terminal 30. The energy is returned thereto via the return terminal 32.

In particular, the RF output stage 28 generates sinusoidal waveforms of high RF energy. The RF output stage 28 is configured to generate a plurality of waveforms having various duty cycles, peak voltages, crest factors, and other suitable parameters. Certain types of waveforms are suitable for specific electrosurgical modes. For instance, the RF output stage 28 generates a 100% duty cycle sinusoidal waveform in cut mode, which is best suited for ablating, fusing and dissecting tissue and a 1-25% duty cycle waveform in coagulation mode, which is best used for cauterizing tissue to stop bleeding.

The generator 20 may include a plurality of connectors to accommodate various types of electrosurgical instruments (e.g., instrument 2, electrosurgical forceps 10, etc.). Further, the generator 20 is configured to operate in a variety of modes such as ablation, monopolar and bipolar cutting coagulation, etc. It is envisioned that the generator 20 may include a switching mechanism (e.g., relays) to switch the supply of RF energy between the connectors, such that, for instance, when the instrument 2 is connected to the generator 20, only the monopolar plug receives RF energy.

The controller 24 includes a microprocessor 25 operably connected to a memory 26, which may be volatile type memory (e.g., RAM) and/or non-volatile type memory (e.g., flash media, disk media, etc.). The microprocessor 25 includes an output port that is operably connected to the HVPS 27 and/or RF output stage 28 allowing the microprocessor 25 to control the output of the generator 20 according to either open and/or closed control loop schemes. Those skilled in the art will appreciate that the microprocessor 25 may be substituted by any logic processor (e.g., control circuit) adapted to perform the calculations discussed herein.

A closed loop control scheme is a feedback control loop wherein sensor circuitry 22, which may include a plurality of sensors measuring a variety of tissue and energy properties (e.g., tissue impedance, tissue temperature, output current and/or voltage, voltage and current passing through the tissue, etc.), provides feedback to the controller 24. Such sensors are within the purview of those skilled in the art. The controller 24 then signals the HVPS 27 and/or RF output stage 28, which then adjust DC and/or RF power supply, respectively. The controller 24 also receives input signals from the input controls of the generator 20 or the instrument 2. The controller 24 utilizes the input signals to adjust power outputted by the generator 20 and/or performs other control functions thereon.

The present disclosure provides for a system and method of determining completion of an electrosurgical procedure. In particular, the method may be implemented as an algorithm (e.g., software) executable by an electrosurgical generator. Although the algorithm is discussed with respect to an ablation procedure, the algorithm may be adapted for any type of electrosurgical procedures, systems and/or methods.

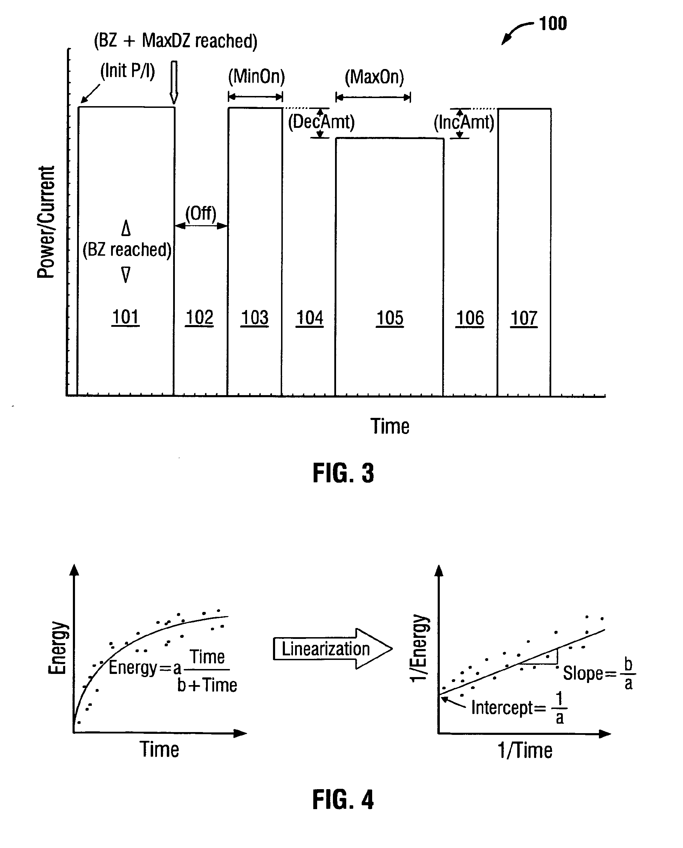

During ablation, energy is applied as an electrosurgical waveform in a pulsatile manner, e.g., in a plurality of cycles, as shown in Fig. 3 for a predetermined period of time (e.g., procedure period) and/or until other termination criteria are met as discussed in more detail below. In particular, Fig. 3 shows a plot 100 of energy applied during ablation versus time. Energy may be delivered at any suitable frequency from about 10 kHz to about 1,000 kHz, in embodiments, from about 400 kHz to about 600 kHz. In embodiments, energy may be delieverd at microwave frequencies from about 300 MHz to about 10,000 MHz. During the first pulse 101, impedance at the tissue-electrode is measured to obtain a baseline impedance (BZ) as energy is applied at an initial power level (P). The energy is delivered until impedance rises above a predetermined threshold (MaxBZ) above the baseline impedance. In embodiments, the threshold may be from about 10 $\Omega$ to about 50 $\Omega$, in embodiments, from about 20 $\Omega$ to about 30 $\Omega$. The baseline impedance may be measured at about 10 seconds into the procedure. Once the threshold impedance is reached (e.g., baseline + threshold), the energy is turned off for a predetermined period of time 102. The algorithm then applies energy in subsequent pulses (e.g., pulses 103, 105, 107) separated by off periods (e.g., periods 104 and 106) until termination criteria (e.g., expiration of time) are reached.

Each of the pulses 101, 103, 105, 107 are applied until the threshold impedance is reached. The energy supplied by the subsequent pulses 103, 105, and 107 is adjusted (e.g., decremented or incremented) based on various variables. In particular, the algorithm also includes a decrement feature that decreases the power when the pulse length of a preceding pulse (e.g., pulse 103) is less than or equal to a predetermined minimum on-time value (MinOn) and an increment feature that increases power if the pulse exceeds a predetermined maximum on-time value (MaxOn). With respect to Fig. 3, the pulse 103 is applied for a time that his shorter than the minimum on-time value, in response to which, the power of the subsequent pulse 105 is decreased by a predetermined power increment (DecAmt). Since the pulse 105 is applied for a period of time longer than the maximum on-time value, in response to which, the power of the subsequent pulse 107 is increased accordingly by a predetermined power increment (IncAmt).

In addition to terminating ablation after expiration of the procedure period the present disclosure provides for an algorithm for terminating ablation as a function of a predicted ablation size. The rate of growth for every ablation is different at any given time, with some ablations completing before the designated procedure duration and others requiring more time. The algorithm of the present disclosure utilizes energy and time to determine when an ablation size is no longer growing as fast as the predetermined rate. When the rate of growth of the ablation size reaches the predetermined threshold, the algorithm alerts the user of completion of ablation.

Energy applied to tissue during a predetermined time period may be correlated with the resulting ablation size, since time and energy have a strong relationship to the rate of growth. The relationship may be defined by correlating ablation data. Although the correlation between time and energy and the growth rate is not linear or of a quadratic/cubic nature, a linearization may be applied to the data. It was observed that correlation between time and energy and size is observed after about 90 seconds from commencement of application of energy. Specifically, a saturation growth rate may be used to linearize the relationship between time/energy and size as shown in Fig. 4. After the transformation was applied, linear regression may be used to determine the relationship between the input parameters and ablation size. Regression may be performed on several subsets of the data using formula (I) below, which defines the relationship between ablation size and time and energy, in which a, b, and c are constants. Constants a, b, and c represent linearization slopes as shown in Fig. 4 that were derived to fit the measured values, which are shown as dots, with the proposed functions.

$$(I) \qquad Size = \cfrac{1}{a + b\cfrac{1}{Time} + c\cfrac{1}{Energy}}$$

In formula (I), estimated size is calculated as an inverse of a sum of inverses of the measured time and the calculated energy. Once size is determined, the growth rate may be calculated using formula (II):

$$(II) \qquad GrowthRate = \frac{Size(i) - Size(i-1)}{Time(i) - Time(i-1)}$$

The growth rate is obtained by differentiation of size and time.

The method according to the present disclosure utilizes energy and time to determine when an ablation volume is no longer growing father than a predetermined growth rate. Once the threshold is reached, the algorithm alerts the user and/or terminates the procedure. The method determines the sizes of the ablation based on the formulas (I) and (II). If ablation energy is applied in a pulsatile manner as discussed above with respect to Fig. 3, pulsing generates discontinuities in the energy curve. This may result in false information to creep into growth rate calculations. To compensate for the pulsing, energy may be summed over longer periods of time, such as the length of an entire energy pulse or about 120 seconds.

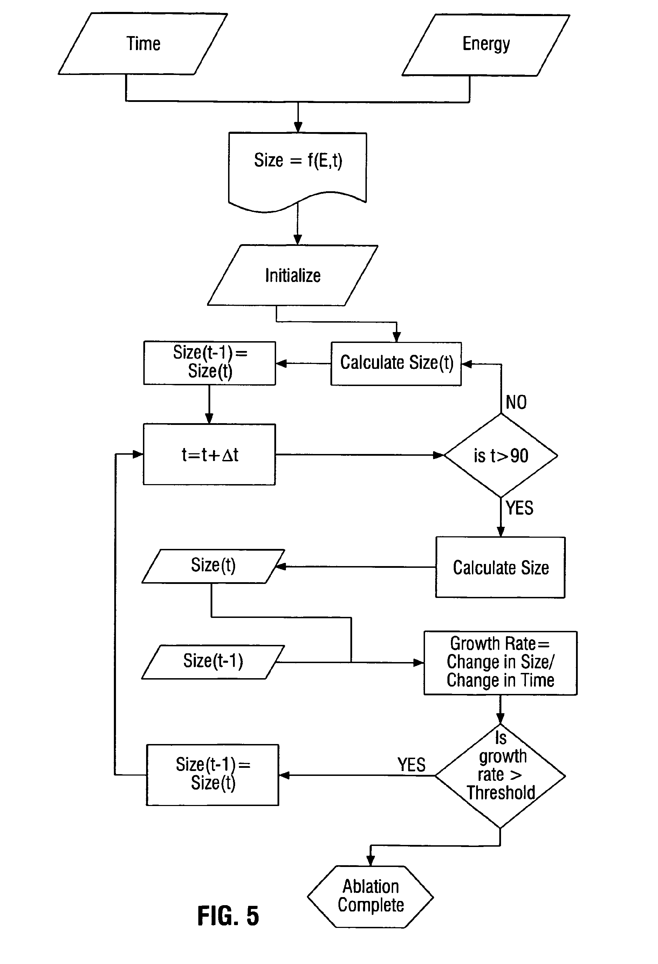

Fig. 5 shows a method for determining ablation completion based on time and energy, which are measured by the generator 20. Energy is summed during application of the energy pulses or a predetermined time period to compensate for the pulsing of energy. Energy may be calculated based on average power (e.g., using voltage and current measurements) and time. The algorithm is initialized and the formulas for calculating the size and growth rate are preloaded. Current size is calculated based on the preloaded formulas which are based on a statistically derived relationship between energy, time and size, as discussed above.

The current size is also saved as previous size and time is incremented by a desired interval. Current time is then compared with an initial time threshold corresponding to the point of time at which energy, time and size begin correlating. The algorithm utilizes a period of 90 seconds. In embodiments, the period may be any suitable interval selected based on a variety of tissue and energy parameters.

Once the initial period of time has expired, the algorithm begins to calculate and compare the growth rate. In particular, the method calculates the size of the ablation volume and saves the value as the current size. The current size is then used in conjunction with the previously calculated size to determine the growth rate via differentiation. If the growth rate is below the predetermined threshold, the current size is saved as previous size and the method returns to the time incrementation step to repeat the size and growth rate calculations. If the growth rate is above the threshold, the method deems the ablation to be complete, at which point the generator 20 may issue an alarm and/or terminate the energy supply.

In addition to time and energy, other tissue and/or energy properties may be utilized to predict ablation size and rate of growth. Temperature has also been shown to correlate well with size estimation. Temperature may be collected at the treatment site (e.g., within tissue) by one or more temperature probes disposed in the vicinity of the electrode or by sensors disposed on the electrosurgical instruments. In addition to temperature, location of the temperature sensors and/or probes is also provided to the generator 20. Location of the temperature sensors and/or probes may be determined using various imaging techniques such as MRI, CT scan, ultrasound and the like. In embodiments, location of the probes may be estimated visually and input into the generator 20.

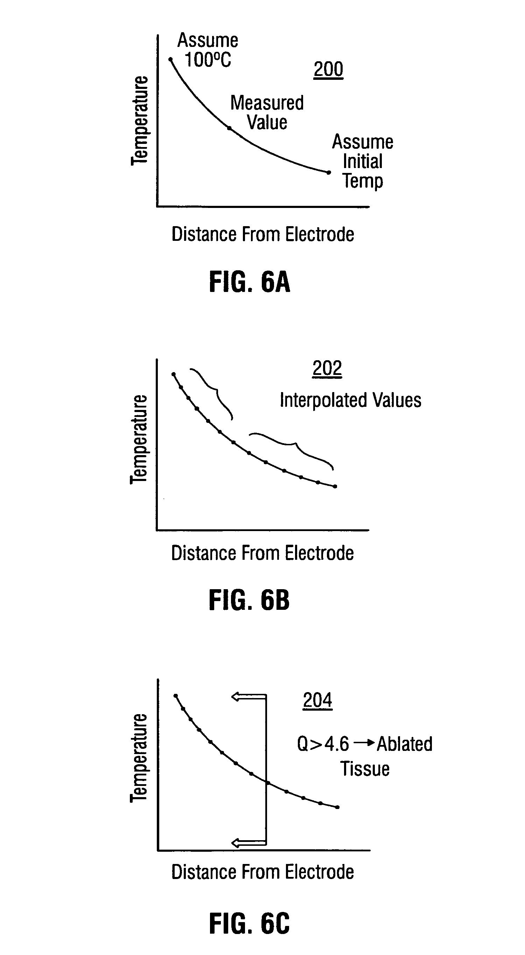

Correlation of temperature and ablation size is shown in plots 200, 202 and 204 of Figs. 6A-C, respectively. Plot 200 shows a temperature graph with boundary conditions applied to the temperature measurements. Boundary conditions represent the outer edges of the ablation volume, namely, normal state of the tissue unaffected by application of energy. Plot 202 shows interpolated temperature values based on measured temperature values. Plot 204 shows calculation of the ablation size using a damage integral formula (III).

$$\text{(III)} \quad \Omega = -\ln\left(\frac{C(\tau)}{C(0)}\right) = A\int_0^\tau e^{\left(\frac{E}{RT(t)}\right)}dt$$

In formula (III), E is a constant derived to fit the measured values to the proposed growth function, R is an ideal gas constant, $T(t)$ is temperature as a function of time variable, t, $C(0)$ is initial concentration, and $C_{(\tau)}$ is concentration as a function of specific time, $\tau$. The plots 200, 202 and 204 visualize the method for correlating the temperature with distance from electrodes and utilize a logarithmic fit to approximate the temperature field. The damage integral is used to estimate the damage done to the tissue.

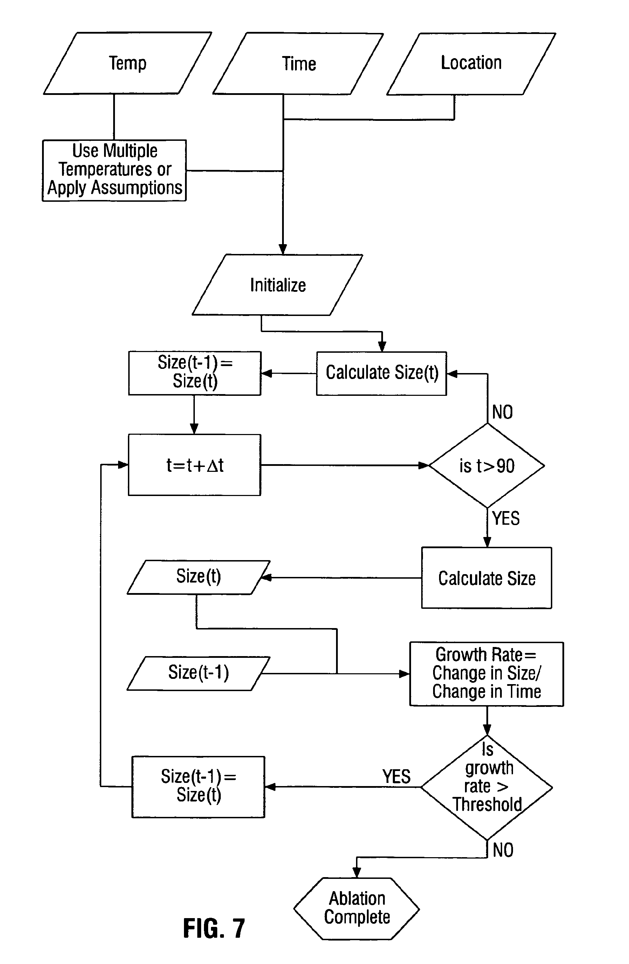

Fig. 7 shows a method for determining ablation completion based on time and temperature, which are measured by the generator 20. The algorithm is initialized and the formulas for calculating the size and growth rate are preloaded. Current size is calculated using a rate type calculation (e.g., first order rate calculation) based on the preloaded formulas which are based on the plots of Figs 6A-C and formula (III), as discussed above.

The current size is also saved as previous size and time is incremented by a desired interval. Current time is then compared with an initial time threshold corresponding to the point of time at which temperature, time and size begin correlating. The algorithm utilizes a period of 90 seconds. In embodiments, the period may be any suitable interval selected based on a variety of tissue and energy parameters.

Once the initial period of time has expired, the algorithm begins to calculate and compare the growth rate. In particular, the method calculates the size of the ablation volume and saves the value as the current size. The current size is then used in conjunction with the previously calculated size to determine the growth rate via differentiation. If the growth rate is below the predetermined threshold, the current size is saved as previous size and the method returns to the time incrementation step to repeat the size and growth rate calculations. If the growth rate is above the threshold, the method deems the ablation to be complete, at which point the generator 20 may issue an alarm and/or terminate the energy supply.

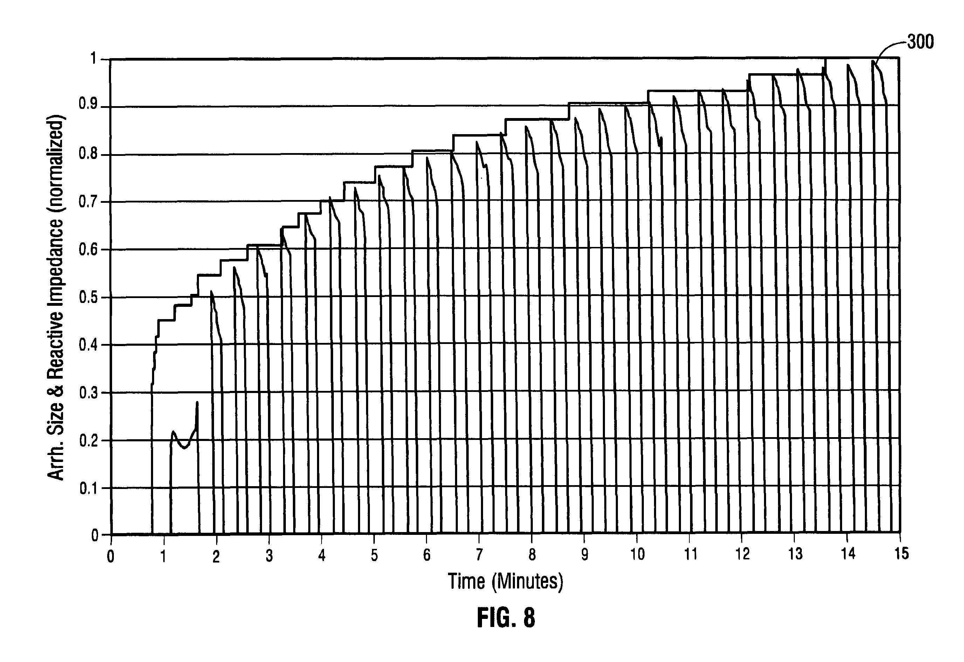

In embodiments, reactive impedance may also be utilized to determine the ablation size and the growth rate thereof and utilize these values to control the energy delivery. In particular, reactive impedance also correlates with the ablation size, which may then be used to determine the growth rate of the ablation volume. As shown in Fig. 8, reactive (e.g., imaginary) impedance response of the tissue also tracks pulsatile nature of the ablation procedure as detailed above. More specifically, Fig. 8 shows a plot 300 of the reactive impedance having a plurality of peaks corresponding to the application of energy during on-time pulses. The peaks of the reactive impedance may be utilized as a parameter for determining completion of ablation.

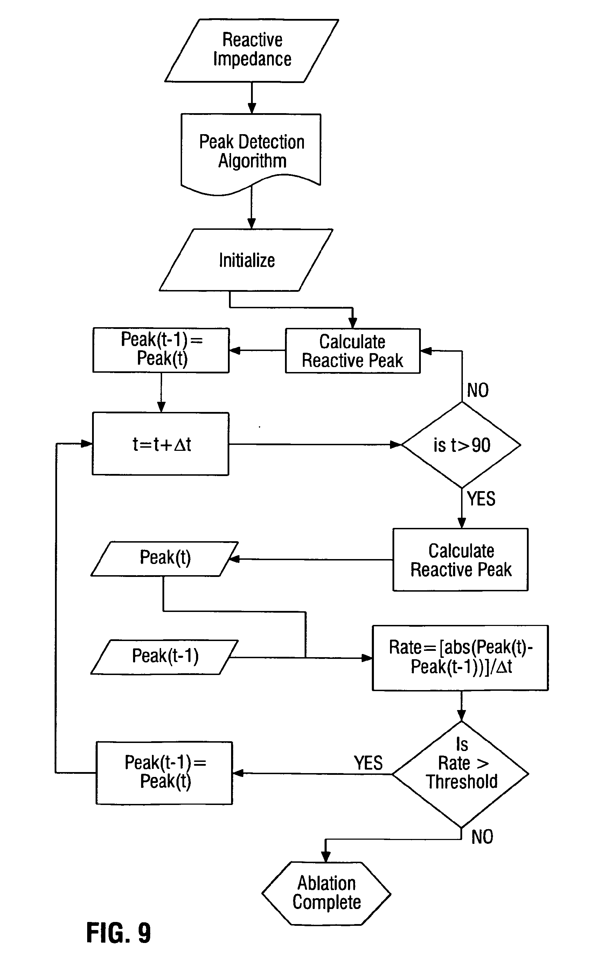

Fig. 9 shows a method for determining ablation completion based on reactive impedance, which are measured by the generator 20. The peaks of the reactive impedance plot are detected by filtering or using a peak detection algorithm as described in commonly-owned U.S. Patent No. 2012/0265194 A1 entitled "System And Method For Monitoring And Intelligent Shut-Off" and U.S. Patent No. 2010/0179538 A1 entitled "And Imaqinary Impedance Process Monitoring And Intelligent Shut-Off".

Once the algorithm is initialized, the peaks of the reactive impedance are determined. The current peak value is also saved as previous peak value and time is incremented by a desired interval. Current time is then compared with an initial time threshold corresponding to the point of time at which reactive impedance and size begin correlating. The algorithm utilizes a period of 90 seconds. In embodiments, the period may be any suitable interval selected based on a variety of tissue and energy parameters.

Once the initial period of time has expired, the algorithm begins to calculate and compare the growth rate. In particular, the method calculates the peaks of the reactive impedance and saves the peak value as the current peak value. The current peak value is then used in conjunction with the previously calculated peak value to determine the growth rate. The growth rate is calculated as the difference in successive peaks divided by the time period between the peaks. If the growth rate is below the predetermined threshold, the current size is saved as previous size and the method returns to the time incrimination step to repeat the growth rate calculations. If the growth rate is above the threshold, the method deems the ablation to be complete, at which point the generator 20 may issue an alarm and/or terminate the energy supply.

While several embodiments of the disclosure have been shown in the drawings and/or discussed herein, it is not intended that the disclosure be limited thereto. Therefore, the above description should not be construed as limiting, but merely as exemplifications of particular embodiments. Those skilled in the art will envision other modifications.

**Claims**

1.   An electrosurgical generator (20), comprising:

   sensor circuitry (22) configured to measure voltage and current delivered to tissue; and
   a controller (24) configured to measure time of energy delivery to tissue and to calculate energy delivered to tissue,
   the controller (24) further configured to estimate a size of an ablation volume and to calculate a growth rate of the ablation volume based on the estimated size,
   **characterized in that** the controller (24) is configured to estimate the size of the ablation volume as a function of the energy delivered to the tissue calculated by the controller (24) and the time of energy delivery to tissue measured by the controller (24).

2.   The electrosurgical generator according to claim 1,
   wherein the controller (24) is further configured to compare the calculated growth rate to a threshold growth rate.

3.   The electrosurgical generator according to claim 2,
   wherein the controller (24) is configured to perform an action in response to a comparison of the calculated growth rate to the threshold growth rate, the action selected from the group consisting of terminating supply of energy to tissue and issuing an alarm.

4.   The electrosurgical generator according to claim 1,

wherein the controller (24) is configured to calculate the growth rate based on differentiation of a plurality of estimated sizes.

5. The electrosurgical generator according to claim 1,
   wherein the controller (24) is configured to calculate the estimated size as an inverse of a sum of inverses of the measured time and the calculated energy.


**Patentansprüche**

1. Elektrochirurgischer Generator (20) mit:

   einer Sensorschaltung (22), die eingerichtet ist, eine Spannung und einen einem Gewebe zugeführent Strom zu messen; und
   einer Steuerung (24), die eingerichtet ist, eine Energiezuführzeit zu Gewebe messen und einem Gewebe zugeführte Energie zu berechnen,
   wobei die Steuerung (24) ferner eingerichtet ist, eine Größe eines Ablationsvolumens abzuschätzen und eine Wachstumsrate des Ablationsvolumens basierend auf der abgeschätzten Größe zu berechnen,
   **dadurch gekennzeichnet, dass** die Steuerung (24) eingerichtet ist, die Größe des Ablationsvolumens in Abhängigkeit der dem Gewebe zugeführten Energie, die durch die Steuerung (24) berechnet wird, und die durch die Steuerung (24) gemessene Energiezuführzeit zu dem Gewebe abzuschätzen.

2. Elektrochirurgischer Generator nach Anspruch 1,
   bei dem die Steuerung (24) ferner eingerichtet ist, die berechnete Wachstumsrate mit einer Grenzwachstumsrate zu vergleichen.

3. Elektrochirurgischer Generator nach Anspruch 2,
   bei dem die Steuerung (24) eingerichtet ist, einen Vorgang als Antwort auf einen Vergleich der berechneten Wachstumsrate mit der Grenzwachstumsrate auszuführen, wobei der Vorgang aus der Gruppe ausgewählt ist, die aus dem Beenden einer Energiezufuhr zu dem Gewebe und einem Ausgeben eines Alarms besteht.

4. Elektrochirurgischer Generator nach Anspruch 1,
   bei dem die Steuerung (24) eingerichtet ist, die Wachstumsrate basierend auf einer Ableitung einer Vielzahl abgeschätzter Größen zu berechnen.

5. Elektrochirurgischer Generator nach Anspruch 1,
   bei dem die Steuerung (24) eingerichtet ist, die abgeschätzte Größe als eine Umkehrfunktion einer Summe von Umkehrfunktionen der gemessenen Zeit und der berechneten Energie zu berechnen.


**Revendications**

1. Générateur électro-chirurgical (20), comprenant :

   un circuit de capteur (22) configuré pour mesurer la tension et le courant délivrés au tissu ; et
   un dispositif de commande (24) configuré pour mesure le temps de la délivrance d'énergie au tissu et pour calculer l'énergie délivrée au tissu,
   le dispositif de commande (24) étant en outre configuré pour estimer une taille d'un volume d'ablation et pour calculer un taux de croissance du volume d'ablation basé sur la taille estimée,
   **caractérisé en ce que** le dispositif de commande (24) est configuré pour estimer la taille du volume d'ablation comme une fonction de l'énergie délivrée au tissu calculée par le dispositif de commande (24) et le temps de la délivrance d'énergie au tissu mesuré par le dispositif de commande (24).

2. Générateur électro-chirurgical selon la revendication 1, dans lequel le dispositif de commande (24) est en outre configuré pour comparer le taux de croissance calculé avec un taux de croissance de seuil.

3. Générateur électro-chirurgical selon la revendication 2, dans lequel le dispositif de commande (24) est configuré pour exécuter une action en réponse à une comparaison du taux de croissance calculé avec le taux de croissance de seuil, l'action sélectionnée dans le groupe consistant à terminer l'amenée de l'énergie au tissu et à émettre une alarme.

4. Générateur électro-chirurgical selon la revendication 1, dans lequel le dispositif de commande (24) est configuré pour calculer le taux de croissance basé sur une différentiation d'une pluralité de tailles estimées.

5. Générateur électro-chirurgical selon la revendication 1, dans lequel le dispositif de commande (24) est configuré pour calculer la taille estimée comme un inverse d'une somme d'inverses du temps mesuré et de l'énergie calculée.

**FIG. 1A**

**FIG. 1B**

**FIG. 2**

EP 2 499 982 B1

FIG. 3

FIG. 4

Time

Energy

Size = f(E,t)

Initialize

Size(t-1) = Size(t)

Calculate Size(t)

NO

t=t+Δt

is t>90

YES

Size(t)

Calculate Size

Size(t-1)

Growth Rate = Change in Size/ Change in Time

Is growth rate > Threshold

Size(t-1) = Size(t)

YES

Ablation Complete

**FIG. 5**

Temperature

Assume
100°C

200

Measured
Value

Assume
Initial
Temp

Distance From Electrode

## FIG. 6A

Temperature

202
Interpolated Values

Distance From Electrode

## FIG. 6B

Temperature

204

Q>4.6 → Ablated
Tissue

Distance From Electrode

## FIG. 6C

**FIG. 7**

**FIG. 8**

EP 2 499 982 B1

```
                        ┌─────────────────┐
                       /   Reactive      /
                      /   Impedance     /
                     └─────────────────┘
                              │
                              ▼
                      ┌─────────────────┐
                      │ Peak Detection  │
                      │   Algorithm     │
                      └─────────────────┘
                              │
                              ▼
                        ┌─────────────┐
                       /  Initialize /
                      └─────────────┘
                              │
                              │
  ┌──────────────┐      ┌─────────────┐
  │ Peak(t-1)=   │◄─────│  Calculate  │◄──────┐
  │  Peak(t)     │      │Reactive Peak│       │
  └──────────────┘      └─────────────┘       │
         │                                    │ NO
         ▼                                    │
  ┌──────────────┐                      ┌──────────┐
  │  t=t+Δt      │─────────────────────►│ is t>90  │
  └──────────────┘                      └──────────┘
         ▲                                    │ YES
         │                                    ▼
         │                              ┌─────────────┐
         │                              │  Calculate  │
  ┌─────────────┐                       │Reactive Peak│
 /  Peak(t)    /◄───────────────────────└─────────────┘
 └─────────────┘
         │
         │
  ┌─────────────┐                  ┌──────────────────┐
 /  Peak(t-1)  /──────────────────►│ Rate=[abs(Peak(t)-│
 └─────────────┘                   │   Peak(t-1))]/Δt  │
         │                         └──────────────────┘
         │                                    │
         │                                    ▼
  ┌──────────────┐                      ┌──────────┐
  │ Peak(t-1)=   │◄───────YES───────────│   Is     │
  │  Peak(t)     │                      │ Rate >   │
  └──────────────┘                      │Threshold │
                                        └──────────┘
                                             │ NO
                                             ▼
                                    ┌──────────────┐
                                    │  Ablation    │
                                    │  Complete    │
                                    └──────────────┘
```

**FIG. 9**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6962587 B
- US 6575969 B
- US 20120265194 A1
- US 20100179538 A1